# EUROPÄISCHE PATENTANMELDUNG

(11) **EP 2 698 131 A2**
(43) Veröffentlichungstag der Anmeldung: **19.02.2014**
(21) Anmeldenummer: 13003763.3
(22) Anmeldetag: 29.07.2013
(51) Int. Cl.: A61F 5/01, A61F 13/06, A61F 13/51

(54) **Orthese**

(30) Priorität: 17.08.2012 DE 102012016271
(71) Anmelder: Otto Bock HealthCare GmbH, 37115 Duderstadt (DE)
(72) Erfinder: Schmitt, Alexander, 34266 Niestetal (DE)
(74) Vertreter: Friedrich, Andreas

(57) **Zusammenfassung**

Die Erfindung betrifft eine Orthese (1) mit wenigstens einem Befestigungsband (10) zum Festlegen der Orthese an einem Körper des Trägers, wobei das Befestigungsband (10)
- eine Innenseite (22), die im festgelegten Zustand der Orthese dem Körper des Trägers zugewandt ist, und
- eine Außenseite (18) aufweist, die im festgelegten Zustand der Orthese von dem Körper des Patienten abgewandt ist,
dadurch gekennzeichnet, dass die Außenseite (18) des Befestigungsbandes (10) eine Beschichtung (16) aufweist, durch die eine Haftung zwischen dem Befestigungsband (10) und einem über der Orthese getragenen Kleidungsstück verstärkt wird.

## Beschreibung

Die Erfindung betrifft eine Orthese mit wenigstens einem Befestigungsband zum Festlegen der Orthese an einem Körper des Trägers, wobei das Befestigungsband eine Innenseite, die im festgelegten Zustand der Orthese dem Körper des Trägers zugewandt ist, und eine Außenseite aufweist, die im festgelegten Zustand der Orthese von dem Körper des Trägers abgewandt ist.

Derartige Orthesen sind aus dem Stand der Technik seit langem bekannt. Sie können beispielsweise als Sprunggelenkorthesen ausgebildet sein und umfassen dann zumeist einen Orthesenkörper, der an dem oder um das Fußgelenk des Trägers gelegt wird.

Aus der DE 698 15 855 T2 ist beispielsweise eine derartige Sprunggelenkstütze bekannt, die über ein flexibles Stützelement verfügt, das über einen an der Innenseite des Knöchels und einen an der Außenseite des Knöchels anzulegenden Anteil verfügt. Ähnliches ist aus der US 5,951,504 und US 5,445,602 bekannt. Die Stützelemente, die den Knöchel des Trägers stützen sollen, bestehen meist aus einem starren Kunststoff oder einem Kohlefaserwerkstoff. Sie sind mit flexiblen und elastischen Materialien gepolstert, um einen besseren Tragekomfort zu gewährleisten.

Andere Arten der Sprunggelenkorthesen umfassen einen festen aber elastischen Orthesenkörper, der ebenfalls um das Fußgelenk des Trägers herum angeordnet wird. Allen Sprunggelenkorthesen ist gemein, dass sie mit wenigstens einem Befestigungsband ausgestattet sind, das zum Festlegen der Sprunggelenkorthese an dem Körper des Trägers gedacht ist. Auch andere Arten von Orthesen werden mittels derartiger Befestigungsbänder am Körper des Trägers festgelegt. Dabei verfügt es beispielsweise im Endbereich des Befestigungsbandes über ein Befestigungselement, das beispielsweise in Form eines Klettverschlusselementes ausgebildet sein kann. Das wenigstens eine Befestigungsband wird in gewünschter Weise beispielsweise um den Knöchel herum gelegt und verbindet so beispielsweise die beiden Anteile des Orthesenkörpers. Durch das Befestigungsband wird die Sprunggelenkorthese am Knöchel des Trägers festgelegt, so dass sie sich auch während einer Belastung, beispielsweise beim Gehen, nicht verschieben kann. Es ist bekannt, durch die Befestigungsbänder beispielsweise zudem einen Druck auf den Knöchel oder auf andere Körperteile auszuüben, um eine weitere Stabilisierung zu erreichen. Dazu sind die Befestigungsbänder oftmals elastisch ausgebildet. Aber auch inelastische Befestigungsbänder sind aus dem Stand der Technik bekannt.

Durch derartige Orthesen soll in der Regel ein Körperteil des Trägers beispielsweise nach einer Verletzung stabilisiert werden, ohne dass die Bewegungsmöglichkeit des Körperteils vollständig eingeengt wird. Für den Genesungsprozess kann es wichtig sein, eine Bewegung des Körperteiles zu trainieren, wobei jedoch darauf zu achten ist, dass eine Überbelastung vermieden wird. Dies wird durch derartige Orthesen erreicht.

Um eine optimale Stabilisierung beispielsweise des Knöchels zu erreichen, ist oftmals jedoch die Sprunggelenkorthese allein nicht ausreichend. Eine bessere Stabilisierung wird erreicht, wenn über die Sprunggelenkorthese ein Schuh, insbesondere ein Schnürschuh getragen wird. Dies hat eine weitere Stabilisierung des Knöchels und des gesamten Fußes zur Folge. Dennoch tritt bei vielen bekannten Sprunggelenkorthesen der Nachteil auf, dass Träger, die die Sprunggelenkorthese angelegt haben und darüber einen Schuh tragen, ein unsicheres und labiles Tragegefühl verspüren. Insbesondere haben diese Träger den Eindruck, den Schuh zu verlieren und/oder in dem Schuh keinen festen Halt zu haben. Dies hat naturgemäß zur Folge, dass eine normale Belastung durch normales Gehen oder Laufen des Knöchels nicht erreicht wird, da der Träger in diesem Fall das Gefühl hat, den Schuh oder den Halt zu verlieren.

Der Erfindung liegt somit die Aufgabe zugrunde, eine Orthese vorzuschlagen, mit der diese Probleme aus dem Stand der Technik beseitigt werden.

Die Erfindung löst die gestellte Aufgabe durch eine gattungsgemäße Orthese, die sich dadurch auszeichnet, dass die Außenseite des Befestigungsbandes eine Beschichtung aufweist, durch die eine Haftung zwischen dem Befestigungsband und einem über der Orthese getragenen Kleidungsstück verstärkt wird. Das Befestigungsband wird folglich beim Anlegen der Orthese am Körperteil des Trägers in gewohnter Weise um das Körperteil herum geführt und die Orthese so am Körper des Trägers festgelegt. Dadurch, dass sich auf der Außenseite, die im angelegten Zustand der Orthese vom Körper des Trägers abgewandt ist, eine Haftbeschichtung befindet, wird erreicht, dass ein zusätzlich zu der Orthese getragenes Kleidungsstück relativ zur Orthese nicht verrutschen kann. Der Träger hat somit ein gewohntes sicheres Tragegefühl und nicht den Eindruck, auf einer besonders rutschigen Oberfläche zu gehen.

Vorzugsweise handelt es sich bei der Orthese um eine Sprunggelenkorthese und bei dem Kleidungsstück, das über der Orthese getragen wird, um einen Schuh. Damit wird insbesondere zur Stabilisierung des Sprunggelenks eine nochmals erhöhte Stabilität erreicht und somit das Sprunggelenk insbesondere bei großer Belastung, wie beispielsweise durch Gehen oder Laufen, zusätzlich gestützt.

Die Beschichtung ist vorteilhafterweise ein thermoplastisches Polymer, insbesondere TPU oder TPE, oder ein Silikon oder Gummi. Diese Materialien haben den Vorteil, dass eine entsprechende Beschichtung leicht auf dem Befestigungsband aufgetragen werden kann und die Materialien zudem kostengünstig und einfach handhabbar sind.

In einer bevorzugten Ausführungsform ist die Außenseite des Befestigungsbandes lediglich abschnittsweise beschichtet. Diese abschnittsweise Beschichtung kann beispielsweise in Form von wenigstens zwei Streifen auf der Außenseite des Befestigungsbandes vorliegen. Auf diese Weise wird erreicht, dass die optimale Haftung zwischen der Außenseite des Befestigungsbandes und damit der Orthese und einem zusätzlich zur Orthese getragenen Kleidungsstück erreicht wird und dennoch Material der Beschichtung eingespart werden kann. Zudem werden gegebenenfalls vorhandene elastische Eigenschaften des Befestigungsbandes selbst nicht durch die Elastizität des aufgebrachten Beschichtungsmaterials verändert.

Die wenigstens zwei Streifen der Beschichtung auf der Außenseite des Befestigungsbandes verlaufen vorteilhafterweise in Längsrichtung des Befestigungsbandes. Natürlich ist auch jede andere Art der abschnittsweisen Beschichtung denkbar. Die Anordnung von wenigstens zwei Streifen parallel zur Längserstreckung des Befestigungsbandes hat jedoch fertigungstechnische Vorteile, da eine derartige Beschichtung besonders einfach auch bei einer Endlos-Fertigung der Bänder aufgebracht werden kann.

Vorzugsweise verfügt die Orthese über mehrere Befestigungsbänder, die jeweils eine Außenseite mit einer zumindest teilweisen Beschichtung aufweisen. Wie bereits dargelegt, umfassen beispielsweise Sprunggelenkorthesen aus dem Stand der Technik zumeist mehrere Befestigungsbänder, um eine optimale Positionierung der Orthese am Sprunggelenk des Trägers zu gewährleisten und gegebenenfalls gleichzeitig an gewünschten Positionen Druck auf den Knöchel des Trägers aufbringen zu können. Entsprechendes ist auch von anderen Orthesen bekannt. Werden nun alle diese Befestigungsbänder mit einer entsprechenden Haftbeschichtung versehen, kann die Haftung zwischen der Orthese und einem über der Orthese getragenen Kleidungsstück weiter verstärkt werden.

In einer bevorzugten Ausführungsform einer Orthese weist auch die Innenseite der Befestigungsbänder eine entsprechende Beschichtung auf. Diese ist vorteilhafterweise aus dem gleichen Material wie die Beschichtung auf der Außenseite des wenigstens einen Befestigungsbandes ausgebildet und kann in diesem Fall besonders einfach im gleichen Fertigungsschritt auf das Befestigungsband aufgebracht werden. Dadurch, dass auch auf der Innenseite des Befestigungsbandes eine Haftbeschichtung vorgesehen ist, wird die Haftung zwischen dem Befestigungsband und beispielsweise einem Orthesenkörper, an dem das Befestigungsband angeordnet werden soll, erhöht, so dass hier eine Haftkraft über die gesamte Fläche oder zumindest der dem beschichteten Anteil der Innenseite entsprechenden Fläche der aneinander anliegenden Teile der Orthese erreicht wird.

Als besonders bevorzugt hat sich dabei herausgestellt, wenn sich die Befestigungsbänder im festgelegten Zustand der Orthese gegenseitig kreuzen. Geschieht dies in einer Ausführungsform, bei der auch auf die Innenseite der Befestigungsbänder eine Beschichtung aufgebracht ist, kommt es im Kreuzungsbereich mehrerer Befestigungsbänder zu einem Kontakt der beiden Haftbeschichtungen auf den beiden Befestigungsbändern. Die Haftbeschichtung auf der Außenseite des Befestigungsbandes, das dem Körper des Trägers am nächsten ist, kommt mit der Beschichtung auf der Innenseite des zweiten, weiter außen verlaufenden, Befestigungsbandes in Kontakt. Es liegen folglich Beschichtungen beispielsweise aus einem thermoplastischem Polymer, Silikon oder Gummi aneinander an, so dass hier eine besonders gute Haftung erreicht wird, wodurch eine besonders gute und sichere Positionierung der Orthese am Körper des Trägers erreicht wird. Sofern beide sich kreuzenden Befestigungsbänder sowohl auf der Innen- als auch auf der Außenseite mit einer Beschichtung versehen sind, ist es für diesen Zweck auch unerheblich, welches der beiden Befestigungsbänder innen, also dem Körper des Trägers zugewandt, oder außen, also näher am Kleidungsstück, angeordnet wird.

Wie bereits dargelegt, besteht die Beschichtung auf der Innenseite und die Beschichtung auf der Außenseite der Befestigungsbänder vorteilhafterweise aus dem gleichen Material, so dass eine besonders einfache, schnelle und kostengünstige Produktion entsprechend beschichteter Bänder möglich ist.

In einer vorteilhaften Ausgestaltung einer Orthese weist zumindest eines der Befestigungsbänder ein Befestigungselement auf, mit dem es an einem Orthesenkörper befestigbar ist, der wenigstens ein korrespondierendes Befestigungselement aufweist. Diese zueinander korrespondierenden Befestigungselemente können beispielsweise zwei Teile eines Klettverschlusses sein. Am Befestigungselement, vorteilhafterweise an dessen Ende, ist ein erstes Klettverschlusselement angeordnet, während an einer dafür vorgesehenen Stelle am Orthesenkörper ein zweites Klettverschlusselement angeordnet ist. Wird nun die Orthese durch das Befestigungsband am Körper des Trägers festgelegt, wird das Befestigungsband in gewohnter Weise um den Körper des Trägers herum geführt. Anschließend werden die beiden korrespondierenden Befestigungselemente aneinander angeordnet.

Durch eine hier beschriebene Orthese wird folglich erreicht, dass die Orthese nicht nur am Körper des Trägers in optimaler Weise festgelegt werden kann, sondern dass zusätzlich auch eine Haftung zwischen der Orthese und einem über der Orthese getragenen Kleidungsstück verstärkt wird, so dass der Tragekomfort insbesondere beispielsweise bei Belastung des Knöchels durch Gehen oder Laufen erhöht wird und der Träger kein Unsicherheitsgefühl verspürt. Dazu müssen die Ausdehnungen der Orthese nicht nennenswert vergrößert werden, so dass die normalerweise vom Träger getragenen Kleidungsstücke in den allermeisten Fällen auch über der Orthese getragen werden können. Dies ist insbesondere bei Schnürschuhen der Fall, die in ihrer Weite durch eine Lockerung der Schnürung einstellbar sind. Zudem ist durch die Schnürung der optimale Druck auf den Knöchel beziehungsweise den Fuß und damit die Sprunggelenkorthese des Trägers einstellbar, so dass hier eine optimale Unterstützung und Stabilisierung des Knöchelgelenks erreicht wird.

Ein erfindungsgemäßes Befestigungsband für eine Orthese zeichnet sich dadurch aus, dass es eine erste Seite und eine zweite Seite aufweist, wobei wenigstens die erste Seite eine Haftbeschichtung aufweist. Vorteilhafterweise sind natürlich sowohl die erste Seite als auch die zweite Seite mit einer entsprechenden Haftbeschichtung versehen.

Mit Hilfe einer Zeichnung wird nachfolgend ein Ausführungsbeispiel der vorliegenden Erfindung näher erläutert. Es zeigt
- Figur 1 -: die Darstellung einer Sprunggelenkorthese gemäß einem ersten Ausführungsbeispiel der vorliegenden Erfindung und
- Figur 2 -: die schematische Darstellung eines Befestigungsbandes in einer Drauf- und einer Schnittdarstellung.

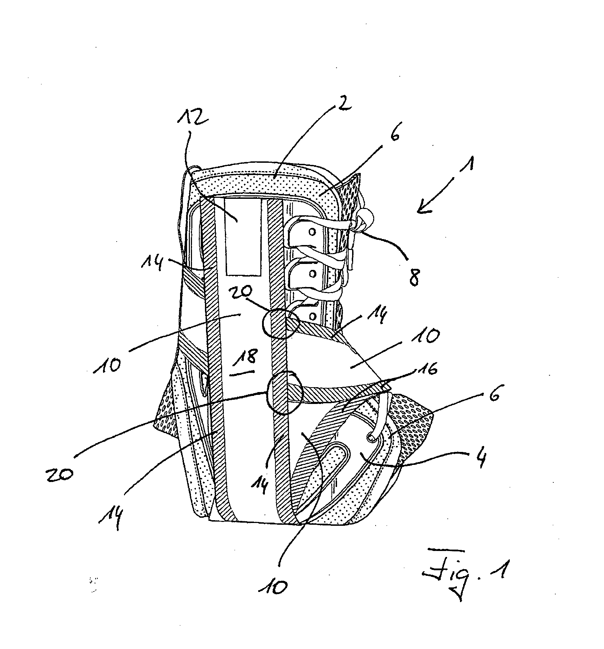

Figur 1 zeigt eine Sprunggelenkorthese 1 gemäß einem ersten Ausführungsbeispiel der vorliegenden Erfindung. Sie umfasst einen Orthesenkörper 2, der am Knöchelbereich eines Trägers angeordnet wird. Der Orthesenkörper 2 weist verstärkte Stützelemente 4 auf, die medial und lateral am Knöchel angeordnet werden. Sie sind zumeist aus einem starren Kunststoff oder beispielsweise einem Kohlefaserverbundwerkstoff hergestellt, können jedoch auch aus einem stabilen Gewebe oder Stoff bestehen. Zum Abpolstern der Stützelemente 4 am Knöchelbereich des Trägers sind Polsterelemente 6 vorgesehen, die ein angenehmes Tragegefühl ermöglichen.

Zur optimalen Anpassung der Sprunggelenkorthese 1 an den Knöchel des Trägers ist eine Schnürung 8 vorgesehen, durch die die Weite der Sprunggelenkorthese 1 individuell einstellbar ist.

Zum Festlegen der Sprunggelenkorthese 1 verfügt die Sprunggelenkorthese 1 über mehrere Befestigungsbänder 10. In Figur 1 ist die Sprunggelenkorthese 1 im festgelegten Zustand dargestellt. Die Befestigungsbänder 10 sind in unterschiedlichen Richtungen um den Knöchelbereich des Trägers herum geführt und verfügen in einem Endabschnitt über ein Befestigungselement 12, das mit einem korrespondierenden, in Figur 1 nicht dargestellten Befestigungselement zusammenwirkt, das am Orthesenkörper 2 angeordnet ist.

Man erkennt, dass die Befestigungsbänder 10 in den Randbereichen über jeweils zwei Streifen 14 verfügen, in denen eine Beschichtung 16 aufgebracht ist, durch die die Haftung zwischen den Befestigungsbändern 10 und einem nicht gezeigten über der Sprunggelenkorthese 1 getragenen Schuh vergrößert wird. Vorzugsweise weisen die Befestigungsbänder 10 nicht nur an der in Figur 1 gezeigten Außenseite 18, sondern auch an einer der Außenseite 18 gegenüberliegenden Innenseite eine entsprechende Beschichtung auf, so dass es in den Kreuzungsbereichen 20, in denen zwei Beschichtungen 16 übereinander liegen, zu einer besonders guten Haftung unterschiedlicher Befestigungsbänder 10 aneinander kommt. Dadurch wird der Halt weiter verstärkt, ohne dass zusätzliche Befestigungs- oder Halteelemente vorgesehen werden müssten.

Durch die in Figur 1 gezeigten Befestigungsbänder 10 ist die Sprunggelenkorthese 1 in optimaler Weise nicht nur am Knöchel des Trägers, sondern auch an einem über der Sprunggelenkorthese 1 getragenen Schuh, der in Figur 1 nicht dargestellt ist, festgelegt.

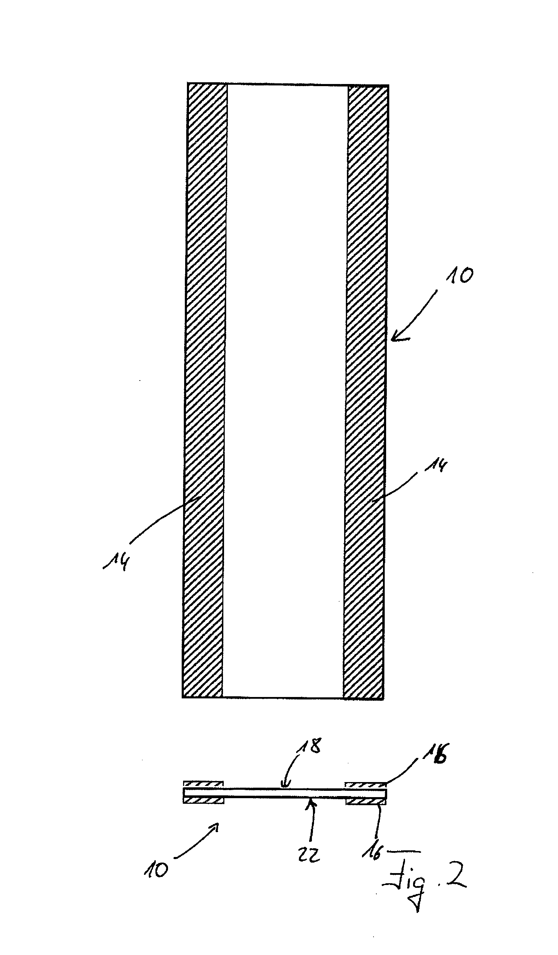

Figur 2 zeigt im oberen Bereich eine schematische Draufsicht und im unteren Bereich eine schematische Schnittdarstellung auf das Befestigungsband 10. Man erkennt in den Randbereichen zwei Streifen 14 einer Beschichtung 16, die, wie im unteren Bereich der Figur 2 dargestellt ist, sowohl auf der Außenseite 18 als auch auf der Außenseite 18 gegenüberliegenden Innenseite 22 angeordnet sind. Die Streifen 14, in denen die Beschichtung 16 aufgebracht ist, verlaufen parallel zu einer Längserstreckung des Befestigungsbandes 10, die sich im oberen Bereich der Figur 2 von unten nach oben erstreckt. Auf diese Weise können die Befestigungsbänder 10 beispielsweise endlos hergestellt und anschließend auf die gewünschte Länge zugeschnitten werden. Dadurch ist es möglich, die Bänder einfach, kostengünstig und schnell herzustellen und dennoch unterschiedliche Anwendungsbereiche und unterschiedliche Orthesengrößen und -formen abdecken zu können.

### Bezugszeichenliste

- 1: Sprunggelenkorthese
- 2: Orthesenkörper
- 4: Stützelement
- 6: Polsterelement
- 8: Schnürung
- 10: Befestigungsband
- 12: Befestigungselement
- 14: Streifen
- 16: Beschichtung
- 18: Außenseite
- 20: Kreuzungsbereich
- 22: Innenseite

## Patentansprüche

1. Orthese mit
wenigstens einem Befestigungsband (10) zum Festlegen der Orthese an einem Körper des Trägers, wobei das Befestigungsband (10)
- eine Innenseite (22), die im festgelegten Zustand der Orthese dem Körper des Trägers zugewandt ist, und
- eine Außenseite (18) aufweist, die im festgelegten Zustand der Orthese von dem Körper des Trägers abgewandt ist,
**dadurch gekennzeichnet, dass** die Außenseite (18) des Befestigungsbandes (10) eine Beschichtung (16) aufweist, durch die eine Haftung zwischen dem Befestigungsband (10) und einem über der Orthese getragenen Kleidungsstück verstärkt wird.

2. Orthese nach Anspruch 1, **dadurch gekennzeichnet, dass** die Orthese eine Sprunggelenkorthese (1) ist und das Kleidungsstück ein Schuh ist.

3. Orthese nach Anspruch 1 oder 2, **dadurch gekennzeichnet, dass** die Beschichtung (16) ein thermoplastisches Polymer, insbesondere TPU oder TPE, oder ein Silikon oder Gummi ist.

4. Orthese nach Anspruch 1, 2 oder 3, **dadurch gekennzeichnet, dass** die Außenseite (18) des Befestigungsbandes (10) nur abschnittsweise beschichtet ist.

5. Orthese nach einem der vorstehenden Ansprüche, **dadurch gekennzeichnet, dass** die Beschichtung (16) in wenigstens zwei Streifen (14) auf der Außenseite (18) des Befestigungsbandes (10) vorliegt.

6. Orthese nach einem der vorstehenden Ansprüche, **dadurch gekennzeichnet, dass** die Orthese mehrere Befestigungsbänder (10) aufweist, die jeweils eine Außenseite (18) mit einer zumindest teilweisen Beschichtung (16) aufweisen.

7. Orthese nach einem der vorstehenden Ansprüche, **dadurch gekennzeichnet, dass** auch die Innenseite (22) der Befestigungsbänder (10) eine Beschichtung (16) aufweist.

8. Orthese nach Anspruch 6 oder 7, **dadurch gekennzeichnet, dass** sich die Befestigungsbänder (10) im festgelegten Zustand der Orthese gegenseitig kreuzen.

9. Orthese nach Anspruch 7 oder 8, **dadurch gekennzeichnet, dass** die Beschichtung (16) auf der Innenseite (22) und die Beschichtung (16) auf der Außenseite (18) der Befestigungsbänder (10) aus dem gleichen Material besteht.

10. Orthese (1) nach einem der vorstehenden Ansprüche, **dadurch gekennzeichnet, dass** zumindest eines der Befestigungsbänder (10) ein Befestigungselement (12) aufweist, mit dem es an einem Orthesenkörper (2) befestigbar ist, der wenigstens ein korrespondierendes Befestigungselement (12) aufweist.

11. Befestigungsband (10) für eine Orthese nach einem der vorstehenden Ansprüche, das eine erste Seite und eine zweite Seite aufweist, wobei wenigstens die erste Seite eine Haftbeschichtung aufweist.
